# FASCICULE DE BREVET EUROPEEN

(11) **EP 1 962 730 B1**
(45) Date de publication et mention de la délivrance du brevet: **02.02.2011**
(21) Numéro de dépôt: 06847191.1
(22) Date de dépôt: 20.12.2006
(51) Int. Cl.: A61F 2/44, A61F 2/46, A61B 17/02

(54) **ENSEMBLE COMPRENANT UN IMPLANT DE REMPLACEMENT D'UN CORPS VERTEBRAL ET UN OUTIL DE DISTRACTION DU RACHIS**
BAUGRUPPE MIT EINEM IMPLANTAT ZUR ERSETZUNG EINES WIRBELSÄULENELEMENTS UND WIRBELSÄULENDISTRAKTIONSINSTRUMENT
ASSEMBLY COMPRISING AN IMPLANT FOR REPLACING A VERTEBRAL BODY AND A SPINAL DISTRACTION TOOL

(30) Priorité: 22.12.2005 FR 0513152
(43) Date de publication de la demande: 03.09.2008
(73) Titulaire: Creaspine, 33600 Pessav (FR)
(72) Inventeur: LE HUEC, Jean-Charles, F-33600 Pessac (FR)
(74) Mandataire: Gaillarde, Frédéric F. Ch.
(86) Numéro de dépôt international: PCT/FR2006/051401
(87) Numéro de publication internationale: WO 2007/074295

(56) Documents cités:
- WO-A-00/45751
- WO-A-02/071986
- US-A- 4 747 394
- US-A1- 2004 122 518

## Description

L'invention concerne le domaine de la chirurgie de la colonne vertébrale et, plus particulièrement, la chirurgie consécutive à un traumatisme tel qu'une fracture ou à l'apparition d'une tumeur, qui a nécessité l'ablation au moins partielle d'un corps vertébral.

Lors de la pose d'implants destinés à remplacer un corps vertébral dont l'ablation au moins partielle a été nécessaire, notamment dans la région thoraco-lombaire, il faut d'abord restaurer la lordose naturelle avant d'insérer l'implant. Cela peut être réalisé au moyen d'une pression postérieure manuelle s'opposant à la cyphose locale créée par la corpectomie. Mais cette méthode est dangereuse et peu efficace.

Une autre technique consiste à réaliser une distraction des vertèbres entre lesquelles l'implant doit être inséré. Cela peut être réalisé par l'implantation de vis dans les vertèbres, suivie par un écartement desdites vertèbres au moyen d'un instrument prenant appui sur lesdites vis. Les vis pourront ensuite servir à la fixation d'une instrumentation à tige(s) ou à plaque(s) qui stabilisera l'ensemble.

Si les vis sont des vis pédiculaires postérieures, la chirurgie doit comporter à la fois une approche postérieure, pour l'implantation et l'écartement des vis et une approche antérieure pour la mise en place de l'implant. Il est donc préférable, pour raccourcir la durée de l'opération et simplifier son exécution, d'implanter les vis dans la région antérieure.

Un inconvénient de cette technique est que l'effort de distraction exercé sur les vis risque de détériorer leur ancrage dans l'os, et la qualité de la fixation de l'instrumentation ne pourra être garantie. De plus, comme la distraction n'est réalisée que d'un seul côté, elle tend à créer une scoliose locale. On ne peut ainsi pas obtenir une distraction qui maintiendrait les plateaux vertébraux sensiblement parallèles. Le document US-B-6,648,891, notamment, décrit un dispositif permettant de réaliser une telle distraction (mais, cependant, ne prévoit pas l'utilisation des vis pour la fixation ultérieure de la plaque constituant le dispositif de stabilisation).

On connaît de WO 00/45751 un ensemble d'implant et de distracteur conforme au préambule de la revendication 1 ci-annexée. Un inconvénient de cet ensemble de la technique antérieure consiste dans le frottement exercé par les lames du distracteur sur les plateaux vertébraux adjacents, lors du retrait de ce distracteur après mise en place de l'implant.

Il existe donc un besoin d'un dispositif de distraction des vertèbres qui, d'une part, procurerait une distraction maintenant les plateaux vertébraux sensiblement parallèles, et d'autre part, fonctionnerait de manière à ne pas gêner, voire à favoriser, l'insertion de l'implant, et pourrait être retiré sans altérer cette insertion .

A cet effet, l'invention a pour objet un ensemble comprenant :
a) un implant destiné à remplacer tout ou partie d'un corps vertébral d'une vertèbre lésée, comportant
   - une partie centrale comprenant un greffon osseux et/ou une cage tubulaire à paroi pleine ou perforée ;
   - deux plateaux disposés aux extrémités de ladite partie centrale, dont les faces externes planes sont destinées à venir au contact des plateaux vertébraux des vertèbres saines encadrant la vertèbre lésée et sont chacune pourvues d'une encoche,
      et
b) un dispositif de distraction du rachis,
   cet ensemble étant remarquable en ce que ledit dispositif de distraction comprend :
   - deux lames se faisant face, ayant chacune une face externe plane destinée à venir au contact d'un plateau vertébral d'une vertèbre saine, lesdites lames étant chacune connectées à une crémaillère ;
   - une partie centrale traversée par lesdites crémaillères et renfermant un pignon dont les dents sont au contact desdites crémaillères, de manière à ce qu'une rotation dudit pignon entraîne un rapprochement ou un éloignement relatif desdites lames, et
   - des moyens pour commander ledit pignon en rotation,
      et en ce que lesdites encoches sont chacune adaptées pour recevoir lesdites lames, les largeurs de ces encoches correspondant aux largeurs de ces lames et la profondeur de ces encoches étant supérieure ou égale à l'épaisseur desdites lames.

Suivant d'autres caractéristiques optionnelles de cet ensemble :
- lesdites lames sont chacune reliées auxdites crémaillères par une portion sinueuse, ces portions sinueuses étant configurées de manière à réaliser un décalage entre lesdites lames et ladite partie centrale afin de permettre un accès libre à l'espace postérieur auxdites lames,
- les faces externes des lames sont parallèles,
- les faces externes desdits plateaux sont parallèles,
- les faces externes desdites lames forment un angle correspondant à un angle de lordose ou de cyphose du rachis,
- les faces externes de ses plateaux forment un angle correspondant à une lordose ou à une cyphose du rachis,
- lesdits plateaux comportent des perforations,
- ladite partie centrale et lesdits plateaux forment une seule pièce,
- lesdits plateaux sont rapportés et fixés sur ladite partie centrale,
- lesdits plateaux comportent des trous taraudés pour le passage de vis assurant leur connexion avec ladite partie centrale,
- lesdits moyens de commande comprennent un outil apte à coopérer de manière amovible avec ledit pignon,
- cet ensemble comprend une goupille traversant ledit pignon, permettant la mise en prise de ce pignon avec ledit outil,
- cet ensemble comprend des pans ménagés à une extrémité dudit pignon, permettant la mise en prise de ce pignon avec ledit outil.

La présente invention a également pour objet un ensemble comprenant un dispositif d'assemblage d'un implant conforme à ce qui précède, remarquable en ce qu'il comporte un socle pourvu d'un logement, dont une extrémité est une paroi fixe comportant, sur sa face tournée vers le logement, une traverse horizontale dont la largeur correspond à celle d'une encoche de l'implant, et dont l'autre extrémité est une tige mobile selon l'axe longitudinal du logement comportant, sur sa face tournée vers le logement, une traverse dont la largeur correspond à celle d'une encoche de l'implant, lesdites traverses se faisant face.

La présente invention a également pour objet un semble comprenant un outil, remarquable en ce qu'il comporte une tige munie à l'une de ses extrémités d'une poignée et à l'autre extrémité d'une douille dont l'espace intérieur est conformé pour venir en prise sans rotation possible avec la face postérieure de la partie centrale du dispositif de distraction, ladite tige renfermant une tige intérieure pouvant tourner à la commande autour de son axe longitudinal et être bloquée en rotation et comportant à son extrémité des moyens pour la mettre en prise avec le pignon.

Comme on l'aura compris, l'invention repose essentiellement sur l'utilisation de deux dispositifs :
- un distracteur comportant deux lames sensiblement parallèles et placées en regard l'une de l'autre, dont les faces externes planes sont destinées à venir s'appliquer contre les plateaux vertébraux après la corpectomie ; un système à crémaillère, actionnable par un outil approprié, connecte ces deux lames et permet leur écartement à la commande du chirurgien, tout en maintenant leur parallélisme ;
- et un implant dont la forme générale peut être cylindrique, destiné à remplacer le corps vertébral qui a été au moins partiellement ôté ; cet implant comporte deux éléments d'extrémité, ou plateaux, qui sont reliés l'un à l'autre soit par un greffon osseux qui leur est fixé, soit par une partie tubulaire, telle qu'un tube à paroi pleine ou un grillage tubulaire en titane, pouvant renfermer un greffon osseux.

Les plateaux comportent des encoches qui permettent l'insertion de l'implant entre les lames du distracteur lorsque celui-ci est en place. Grâce à une telle insertion, aucune sur-distraction des plateaux vertébraux n'est nécessaire. En d'autres termes, il est suffisant d'écarter ces plateaux vertébraux l'un de l'autre d'une distance à peine supérieure à la longueur de l'implant, ce qui permet de minimiser l'impact opératoire dans la zone concernée.

Après cette insertion, les lames du distracteur sont rapprochées l'une de l'autre jusqu'à ce que les plateaux vertébraux viennent au contact des surfaces externes des plateaux de l'implant et commencent à mettre celui-ci en compression. Le distracteur est ensuite retiré, cette dernière phase étant facilitée par le fait que la profondeur des encoches de l'implant est supérieure à celle des lames du distracteur: grâce à cette caractéristique, aucun frottement n'est appliqué par les lames du distracteur aux plateaux vertébraux pendant le retrait de ces lames.

L'invention propose également un ensemble comprenant un dispositif de montage permettant l'assemblage de l'implant avant sa mise en place.

L'invention sera mieux comprise à la lecture de la description qui suit, donnée en référence aux figures annexées suivantes :
- les figures 1 et 2 qui montrent en perspective un exemple de dispositif de distraction du rachis selon l'invention, dont les deux lames sont en position rapprochée (fig.1) et en position écartée (fig.2) ;
- la figure 3 qui montre en coupe partielle et en perspective le dispositif de distraction précédent, portant un exemple d'implant selon l'invention, et en coupe partielle et en perspective l'extrémité d'un exemple d'outil pour la mise en rotation du pignon du dispositif de distraction ;
- la figure 4 qui montre en perspective deux exemples (fig.4a et 4b) d'implants selon l'invention ;
- la figure 5 qui montre en perspective un dispositif pour l'assemblage d'un implant selon l'invention ;
- la figure 6 qui montre en perspective un exemple d'outil pour la mise en rotation du pignon du dispositif de distraction, et le dispositif de distraction lui-même ; et
- la figure 7 qui montre un implant selon l'invention en cours d'installation.

Le premier élément de l'instrumentation selon l'invention est un dispositif de distraction ou distracteur 1.

Sa fonction consiste, après que le chirurgien a procédé à une corpectomie sur la vertèbre 51 lésée, à établir entre les plateaux vertébraux de deux vertèbres saines 52, 53 situées au-dessus et au-dessous de la vertèbre lésée 51, une distance qui permettra l'insertion de l'implant 18a, 18b qui sera décrit plus loin.

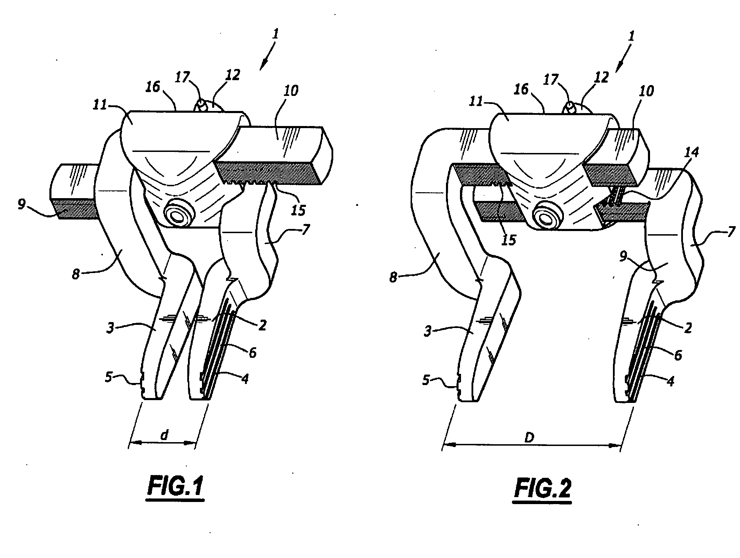

Ce distracteur 1 comporte deux lames 2, 3 comportant chacune une face externe plane 4, 5. Ces faces externes 4, 5 sont celles qui sont destinées à entrer en contact avec les plateaux vertébraux. Elles comportent de préférence des stries 6 procurant un contact rugueux avec les plateaux vertébraux, limitant les possibilités de glissement des lames 2, 3. Lorsque le distracteur 1 est assemblé, elles doivent être maintenues sensiblement parallèles l'une à l'autre (dans l'exemple représenté). A cet effet, les lames 2, 3 sont chacune connectées par une portion sinueuse 7, 8 à une portion rectiligne 9, 10. Ces portions rectilignes sont insérées dans une pièce centrale 11 qui les maintient parallèles l'une à l'autre, orientées selon la direction dans laquelle les lames 2, 3 doivent être écartées. La pièce centrale 11 renferme un pignon 12 dont les dents 13 sont en contact avec des crémaillères 14, 15 ménagées sur les faces des portions rectilignes 9, 10 qui sont tournées vers lui. Une extrémité du pignon 12 dépasse de la face arrière 16 de la pièce centrale 11, et est traversée par une goupille 17 qui permet au chirurgien, à l'aide d'un instrument approprié dont un exemple sera décrit plus loin, de faire tourner le pignon 12 et, ainsi, de rapprocher ou d'écarter à volonté les lames 2, 3 du distracteur 1, entre une position d'écartement minimal « d » (fig.1) et une position d'écartement maximal « D » (fig.2). Les lames 2, 3 ont, par exemple, une longueur de 35mm et une largeur de 7mm, de manière à pouvoir s'étendre en longueur sur la plus grande partie des plateaux vertébraux et à présenter une surface de contact suffisante avec les plateaux vertébraux.

Le deuxième élément de l'instrumentation est un implant 18a, 18b de forme générale cylindrique, pour lequel plusieurs types de configuration sont envisageables.

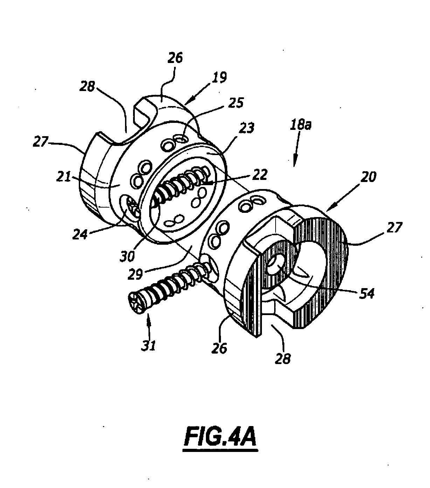

Dans la configuration de la figure 4a, l'implant 18a comporte deux plateaux 19, 20, dont chacun comprend :
- une portion cylindrique 21 présentant une perforation longitudinale 22 sur sa face 23 destinée à être tournée en regard de l'autre plateau 19, 20 de l'implant 18a, et une série de perforations filetées 24, 25 sur sa paroi latérale ;
- et une portion terminale 26 qui comporte une face externe 27 plane destinée à venir au contact d'un plateau vertébral lorsque l'implant 18a est mis en place ; cette face externe 27 comporte également une encoche 28 dont la largeur correspond à la largeur d'une lame 2, 3 du distracteur 1, et dont la hauteur est légèrement supérieure à celle d'une lame 2, 3 du distracteur 1.

L'implant 18a comporte également une partie centrale 29, sensiblement cylindrique dans l'exemple représenté, connectant les plateaux 19, 20, constituée par un greffon osseux. Le greffon est enfoncé dans les perforations longitudinales 22 des plateaux 19, 20 et y est maintenu par des vis 30, 31 pénétrant dans des perforations filetées 24 de grand diamètre ménagés sur les parois latérales des plateaux 19, 20.

Des perforations 54 permettent au greffon osseux de se vasculariser et de traverser les plateaux 19, 20 de part en part pour venir au contact des plateaux vertébraux et réaliser ainsi la fusion osseuse des deux vertèbres saines 52, 53 encadrant la vertèbre lésée 51.

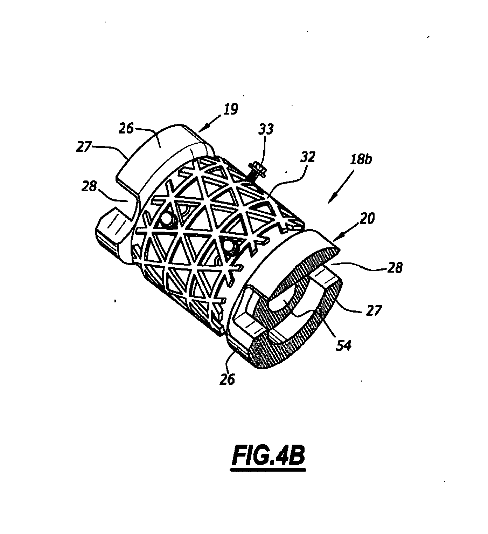

Dans la configuration de la figure 4b, l'implant 18b est semblable au précédent, mis à part que la partie centrale qui relie les plateaux 19, 20 est constituée par un grillage tubulaire 32 en titane (par exemple) renfermant éventuellement un greffon osseux (non représenté). Le grillage 32 est fixé aux plateaux 19, 20 par des vis 33 pénétrant dans des perforations filetées 25 de petit diamètre ménagées sur les parois latérales des plateaux 19, 20.

Si on ne désire pas placer de greffon osseux dans la partie centrale de l'implant, la présence des perforations 54 des plateaux 19, 20 n'est pas nécessaire.

Le grillage tubulaire 32 pourrait être remplacé par un tube à paroi pleine. Cependant, le grillage 32, du fait qu'il présente des perforations, permet la vascularisation du greffon, s'il y en un, et la circulation de fluide.

A noter également que l'on peut envisager, dans une variante non représentée, que la partie centrale 29 et les plateaux 19, 20 de l'implant 18 forment une seule pièce.

Dans l'exemple représenté, les plateaux 19, 20 comportent les deux types de perforations filetées 24, 25, de manière à pouvoir être utilisés indifféremment avec un greffon 29 (fig.4a) ou un grillage tubulaire 32 (fig.4b) ou un tube. Bien entendu, ils pourraient ne comporter qu'un seul type de perforations 24, 25, compatible avec l'une et/ou l'autre de ces variantes de partie centrale.

Pour une utilisation de l'implant 18a, 18b dans la région thoraco-lombaire, les plateaux 19, 20 ont un diamètre de l'ordre de 20mm. Les plans de leurs faces externes 27 peuvent être parallèles une fois l'implant assemblé : les plateaux vertébraux seront alors également parallèles lorsque l'implant sera mis en place.

De préférence, les faces externes 27 des plateaux 19, 20 sont recouvertes d'hydroxyapatite ou d'un matériau similaire stimulant la croissance osseuse, de manière à assurer un meilleur ancrage de l'implant 18a, 18b sur les plateaux vertébraux.

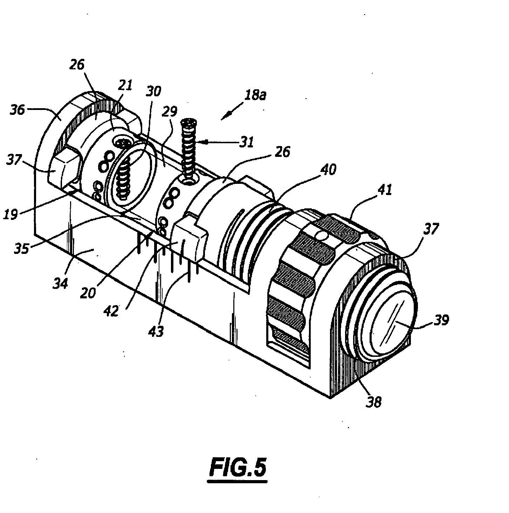

Avantageusement, pour l'assemblage de l'implant 18a, 18b on peut utiliser un dispositif tel que celui représenté sur la figure 5.

Il se compose d'un socle 34 définissant un logement 35 dans lequel on place les différents éléments de l'implant (qui, dans l'exemple représenté, est du type 18a selon la figure 4a). La première des extrémités du socle est une paroi fixe 36 et porte, sur sa face tournée vers le logement 35, une traverse horizontale 37 dont la largeur correspond à celle d'une encoche 28 d'un plateau 19, 20 de l'implant 18a. La seconde extrémité du socle 34 est une paroi 37 comportant une perforation 38 à travers laquelle passe une tige cylindrique 39 portant un filetage externe 40. Une molette filetée 41, maintenue dans le socle 34 et traversée par la tige 39, permet à l'utilisateur de faire avancer ou reculer la tige 39 à l'intérieur du logement 35 du socle 34. A son extrémité tournée vers l'intérieur du socle 34, la tige 39 porte une traverse horizontale 42 qui, comme la traverse 37 de la première extrémité du socle 34, a sa largeur qui correspond à celle d'une encoche 28 d'un plateau 19, 20 de l'implant 18a. Les deux traverses 38, 42 se font donc rigoureusement face.

Lors de la préparation de l'implant 18a, préalablement à son installation, les plateaux 19, 20 sont placés dans le logement 35 de manière à s'insérer sur les traverses 37, 42. On s'assure ainsi qu'après l'assemblage de l'implant 18a, les encoches 28 seront rigoureusement parallèles. Puis on insère un greffon osseux cylindrique 29 dans l'un des plateaux 19, 20, on l'y fixe à l'aide de vis 30, 31, et on réalise le rapprochement des deux plateaux 19, 20 en tournant la molette 41, de façon à réaliser l'insertion du greffon 29 dans l'autre des plateaux 19, 20. Après quoi, le greffon 29 est fixé dans l'autre des plateaux 19, 20 par des vis 30, 31. Des graduations 43 ménagées sur le socle 34 permettent de s'assurer que la longueur totale de l'implant 18a assemblé est bien celle souhaitée par le chirurgien.

Le dispositif d'assemblage de la figure 5 peut aussi, naturellement, être utilisé pour assembler un implant 18b dont la partie centrale cylindrique est constituée par un tube ou un filet tubulaire 32 renfermant un greffon osseux.

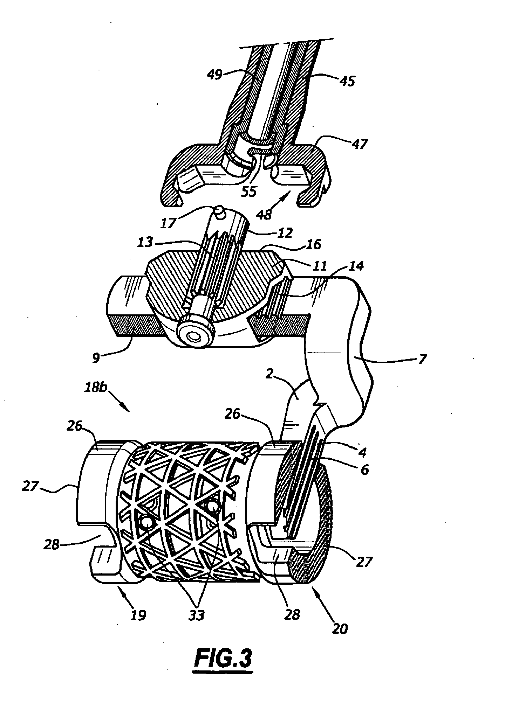

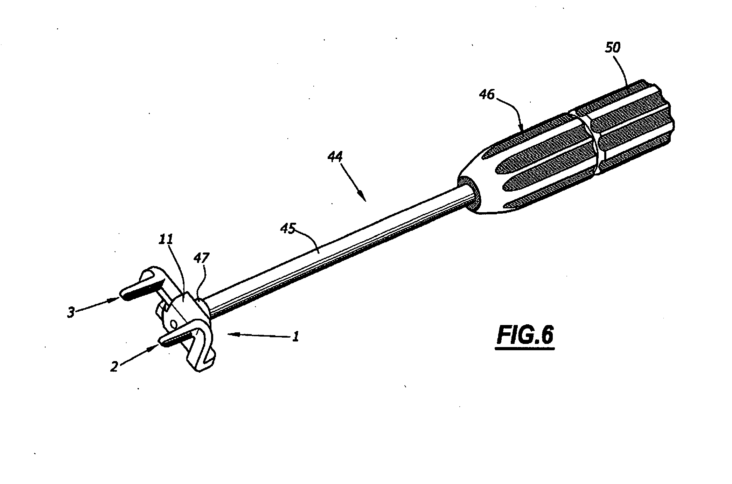

L'instrumentation peut avantageusement être complétée par un outil 44 représenté sur la figure 6, grâce auquel l'écartement des lames 2, 3 du distracteur 1 peut réglé. Il comporte une tige 45 munie à l'une de ses extrémités d'une poignée 46 et à l'autre de ses extrémités d'une douille 47 dont l'espace intérieur 48 est conformé pour que la tige 45 puisse venir en prise sans rotation possible contre la face arrière 16 de la pièce centrale 11 du distracteur 1 (voir figure 3). A l'intérieur de cette tige 45 se trouve une tige intérieure 49 pouvant tourner autour de son axe longitudinal lorsque le chirurgien tourne une poignée 50 placée à la suite de la poignée 46 liée au tube 45.

L'extrémité 55 de la tige intérieure 49 est conformée de façon à pouvoir venir en prise avec la goupille 17 du pignon 12. Lorsque la goupille 17 est saisie par l'extrémité 55 de la tige intérieure 49, une rotation de la poignée 50 liée à la tige intérieure 49 est transmise au pignon 12, ce qui permet de déplacer les lames 2, 3 l'une par rapport à l'autre de manière à les rapprocher ou à les écarter. Des moyens de blocage (non représentés) de la tige intérieure 49 permettent de maintenir l'écart entre les lames 2, 3 à une valeur choisie par le chirurgien.

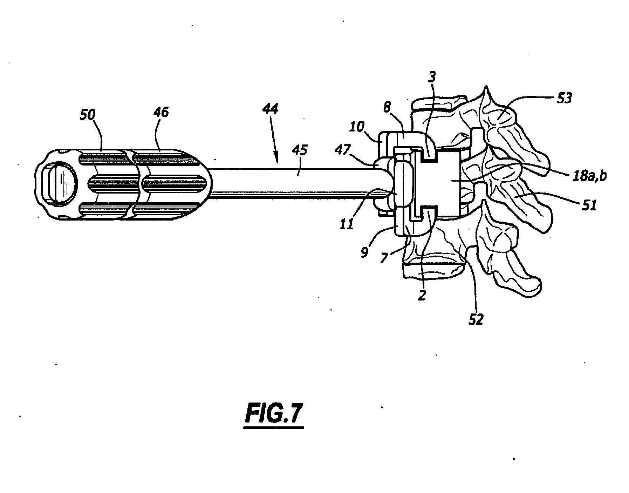

L'invention est utilisée comme exposé dans ce qui suit, et représenté sur la figure 7.

Dans un premier temps, le chirurgien réalise une corpectomie sur la vertèbre lésée 51, de manière à ne plus en laisser subsister que les parties saines en libérant un espace dans lequel l'implant 18a, 18b pourra être inséré.

Ensuite, le chirurgien insère les lames 2, 3 du distracteur 1 entre les plateaux vertébraux des vertèbres saines 52, 53 entourant la vertèbre lésée 51. Puis, à l'aide de l'outil 44 (ou de tout autre instrument fonctionnellement équivalent), il augmente progressivement l'écartement des lames 2, 3 de manière à réaliser une distraction de l'espace intervertébral concerné légèrement supérieure à la hauteur de l'implant. Une fois cette distraction réalisée, le distracteur 1 est bloqué.

Ensuite, le chirurgien insère l'implant 18a, 18b, préalablement préparé, entre les lames 2, 3 du distracteur 1. A cet effet, les encoches 28 des plateaux 19, 20 autorisent un glissement extrêmement facile de l'implant 18a, 18b entre les lames 2, 3. On note également que la configuration des portions sinueuses 7, 8 prolongeant les lames 2, 3 permet avantageusement de libérer l'espace situé à l'arrière des lames 2, 3 pour permettre l'insertion de l'implant 18a, 18b entre les lames 2, 3, la pièce centrale 11 étant décalée angulairement (d'environ 30° par exemple) par rapport à cet espace. De cette façon, l'outil 44 n'interfère pas avec l'implant 18a, 18b. La figure 7 représente ce stade des opérations.

Ensuite, le chirurgien actionne l'outil 44 de façon à réduire l'écart entre les lames 2, 3 jusqu'à ce que les plateaux vertébraux des vertèbres saines 52, 53 viennent au contact des faces externes planes 27 des plateaux 19, 20 de l'implant 18a, 18b. Lorsque cette mise en contact est réalisée, le distracteur 1 est retiré.

Enfin, le chirurgien peut compléter l'opération par la pose d'une instrumentation antérieure ou postérieure de stabilisation de la région du rachis concernée. Cette instrumentation comporte le plus habituellement une ou des tiges longitudinales ou des plaques, fixées sur les vertèbres saines 52, 53 par des éléments d'ancrage osseux (vis ou crochets). Cette instrumentation assure une mise en compression de l'implant 18a, 18b.

Les différents éléments de l'implant 18a, 18b peuvent être réalisés en tout matériau biocompatible apte à supporter les charges axiales auxquelles ils seront soumis : acier inoxydable, titane, fibres de carbone... Le diamètre de l'implant 18a, 18b peut être, par exemple, de 16 à 25mm en fonction de la région du rachis où il sera installé. Un diamètre de 16mm convient en général pour la région thoracique et un diamètre de 25mm pour la région lombaire.

Dans la variante de l'invention représentée sur les figures, les faces externes 27 de l'implant 18a, 18b sont parallèles. Mais il est aussi possible de les rendre convergentes selon un angle de quelques degrés (4° par exemple), de manière à reconstruire une lordose ou une cyphose du rachis après la mise en place de l'implant 18a, 18b. Dans ce cas, la configuration du socle 34 du dispositif d'assemblage doit être modifiée en conséquence. On peut également prévoir un distracteur 1 dont les lames 2, 3 formeraient un angle correspondant.

La forme générale cylindrique de l'implant décrit et représenté n'est qu'un exemple, et n'est pas obligatoire. La forme du dispositif d'assemblage qui a été décrit et représenté peut être adaptée de manière évidente à celle d'un implant non cylindrique.

Les moyens permettant au chirurgien de faire tourner le pignon 12 ne se limitent pas à la goupille 17 qui a été décrite et représentée. Ils pourraient, par exemple, être constitués par des pans ménagés à l'extrémité du pignon, pouvant venir en prise avec une douille qui serait ménagée à l'extrémité de la tige intérieure 49 de l'outil 44 (ou d'un autre outil fonctionnellement équivalent). Il peut, d'ailleurs y avoir à la fois une goupille 17 et des pans, de manière à autoriser l'utilisation de plusieurs configurations d'outils 44 avec un même distracteur 1.

L'invention a notamment pour avantage de nécessiter pour la mise en place de l'implant une chirurgie peu invasive et de procurer une excellente stabilisation du rachis, en particulier lorsqu'elle coopère avec un dispositif de stabilisation à tige(s) ou plaque(s) habituel.

## Revendications

1. Ensemble comprenant :
a) un implant (18a, 18b) destiné à remplacer tout ou partie d'un corps vertébral d'une vertèbre lésée (51), comportant
- une partie centrale comprenant un greffon osseux (29) et/ou une cage tubulaire à paroi pleine ou perforée (32) ;
- deux plateaux (19, 20) disposés aux extrémités de ladite partie centrale, dont les faces externes planes (27) sont destinées à venir au contact des plateaux vertébraux des vertèbres saines (52, 53) encadrant la vertèbre lésée (51) et sont chacune pourvues d'une encoche (28),
et
b) un dispositif de distraction du rachis (1),
cet ensemble étant **caractérisé en ce que** ledit dispositif de distraction comprend :
- deux lames (2, 3) se faisant face, ayant chacune une face externe (5, 6) plane destinée à venir au contact d'un plateau vertébral d'une vertèbre saine (52, 53), lesdites lames (2, 3) étant chacune connectées à une crémaillère (14, 15) ;
- une partie centrale (11) traversée par lesdites crémaillères (14, 15) et renfermant un pignon (12) dont les dents (13) sont au contact desdites crémaillères (14, 15), de manière à ce qu'une rotation dudit pignon (12) entraîne un rapprochement ou un éloignement relatif desdites lames (2, 3), et
- des moyens pour commander ledit pignon (12) en rotation,
et **en ce que** lesdites encoches (28) sont chacune adaptées pour recevoir lesdites lames (2, 3), les largeurs de ces encoches (28) correspondant aux largeurs de ces lames (2, 3) et la profondeur de ces encoches (28) étant supérieure ou égale à l'épaisseur desdites lames (2, 3).

2. Ensemble selon la revendication 1, **caractérisé en ce que** lesdites lames (2, 3) sont chacune reliées auxdites crémaillères (14, 15) par une portion sinueuse (7, 8), ces portions sinueuses (7, 8) étant configurées de manière à réaliser un décalage entre lesdites lames (2, 3) et ladite partie centrale (11) afin de permettre un accès libre à l'espace postérieur auxdites lames (2, 3).

3. Ensemble selon l'une des revendications 1 ou 2, **caractérisé en ce que** les faces externes (5, 6) des lames (2, 3) sont parallèles.

4. Ensemble selon la revendication 3, **caractérisé en ce que** les faces externes (27) desdits plateaux (19, 20) sont parallèles.

5. Ensemble selon l'une des revendications 1 ou 2, **caractérisé en ce que** les faces externes (5, 6) desdites lames (2, 3) forment un angle correspondant à un angle de lordose ou de cyphose du rachis.

6. Ensemble selon la revendication 5, **caractérisé en ce que** les faces externes (27) desdits plateaux (19, 20) forment un angle correspondant à une lordose ou à une cyphose du rachis.

7. Ensemble selon l'une quelconque des revendications précédentes, **caractérisé en ce que** lesdits plateaux (19, 20) comportent des perforations (54).

8. Ensemble selon l'une quelconque des revendications précédentes, **caractérisé en ce que** ladite partie centrale et lesdits plateaux (19, 20) forment une seule pièce.

9. Ensemble selon l'une quelconque des revendications 1 à 7, **caractérisé en ce que** lesdits plateaux (19, 20) sont rapportés et fixés sur ladite partie centrale.

10. Ensemble selon la revendication 9, **caractérisé en ce que** lesdits plateaux (19, 20) comportent des trous taraudés (24, 25) pour le passage de vis assurant leur connexion avec ladite partie centrale.

11. Ensemble selon l'une quelconque des revendications précédentes, **caractérisé en ce que** lesdits moyens de commande comprennent un outil (44) apte à coopérer de manière amovible avec ledit pignon (12).

12. Ensemble selon la revendication 11, **caractérisé en ce qu'**il comprend une goupille (17) traversant ledit pignon (12), permettant la mise en prise de ce pignon (12) avec ledit outil (44).

13. Ensemble selon la revendication 11, **caractérisé en ce qu'**il comprend des pans ménagés à une extrémité dudit pignon (12), permettant la mise en prise de ce pignon (12) avec ledit outil (44).

14. Ensemble conforme à l'une quelconque des revendications 1 à 13, **caractérisé en ce qu'**il comprend un dispositif d'assemblage dudit implant (18a, 18b), ce dispositif comportant un socle (34) pourvu d'un logement (35), dont une extrémité est une paroi fixe (36) comportant, sur sa face tournée vers le logement (35), une traverse horizontale (37) dont la largeur correspond à celle d'une encoche (28) de l'implant (18a, 18b), et dont l'autre extrémité est une tige (39) mobile selon l'axe longitudinal du logement (35) comportant, sur sa face tournée vers le logement (35), une traverse (42) dont la largeur correspond à celle d'une encoche (28) de l'implant (18a, 18b), lesdites traverses (37, 42) se faisant face.

15. Ensemble conforme à l'une quelconque des revendications 11 à 14, **caractérisé en ce qu'**il comprend un outil (44) comportant une tige (45) munie à l'une de ses extrémités d'une poignée (46) et à l'autre extrémité d'une douille (47) dont l'espace intérieur (48) est conformé pour venir en prise sans rotation possible avec la face postérieure (16) de la partie centrale (11) du dispositif de distraction (1), ladite tige (45) renfermant une tige intérieure (49) pouvant tourner à la commande autour de son axe longitudinal et être bloquée en rotation et comportant à son extrémité des moyens (55) pour la mettre en prise avec le pignon (12).

## Claims

1. Assembly comprising:
a) an implant (18a, 18b) intended to replace all or part of a vertebral body of a damaged vertebra (51), including
- a central part comprising a bone graft (29) and/or a tubular cage with a solid or perforated wall (32);
- two plates (19, 20) which are arranged at the ends of said central part and the flat outer faces (27) of which are intended to come into contact with the vertebral endplates of the healthy vertebrae (52, 53) surrounding the damaged vertebra (51) and are each provided with a slot (28),
and
b) a spinal distraction device (1),
this assembly being **characterised in that** said distraction device comprises:
- two blades (2, 3) facing one another, each having a flat outer face (5, 6) intended to come into contact with a vertebral endplate of a healthy vertebra (52, 53), each of said blades (2, 3) being connected to a rack (14, 15);
- a central part (11) which is penetrated by said racks (14, 15) and encloses a pinion (12), the teeth (13) of which are in contact with said racks (14, 15) so that a rotation of said pinion (12) causes said blades (2, 3) to move towards one another or away from one another, and
- means for controlling said pinion (12) in rotation,
and **in that** said slots (28) are each able to receive said blades (2, 3), the widths of these slots (28) corresponding to the widths of these blades (2, 3) and the depth of these slots (28) being greater than or equal to the thickness of said blades (2, 3).

2. Assembly according to claim 1, **characterised in that** said blades (2, 3) are each connected to said racks (14, 15) by a sinuous portion (7, 8), these sinuous portions (7, 8) being configured so as to create an offset between said blades (2, 3) and said central part (11) in order to allow free access to the space posterior to said blades (2, 3).

3. Assembly according to one of claims 1 or 2, **characterised in that** the outer faces (5, 6) of the blades (2, 3) are parallel.

4. Assembly according to claim 3, **characterised in that** the outer faces (27) of said plates (19, 20) are parallel.

5. Assembly according to one of claims 1 or 2, **characterised in that** the outer faces (5, 6) of said blades (2, 3) form an angle corresponding to an angle of lordosis or kyphosis of the spine.

6. Assembly according to claim 5, **characterised in that** the outer faces (27) of said plates (19, 20) form an angle corresponding to a lordosis or kyphosis of the spine.

7. Assembly according to any one of the preceding claims, **characterised in that** said plates (19, 20) comprise perforations (54).

8. Assembly according to any one of the preceding claims, **characterised in that** said central part and said plates (19, 20) form a single piece.

9. Assembly according to any one of claims 1 to 7, **characterised in that** said plates (19, 20) are attached and fixed to said central part.

10. Assembly according to claim 9, **characterised in that** said plates (19, 20) comprise threaded holes (24, 25) for the passage of screws for the connection thereof to said central part.

11. Assembly according to any one of the preceding claims, **characterised in that** said control means comprise a tool (44) which is able to cooperate with said pinion (12) in a removable manner.

12. Assembly according to claim 11, **characterised in that** it comprises a pin (17) passing through said pinion (12), enabling this pinion (12) to engage with said tool (44).

13. Assembly according to claim 11, **characterised in that** it comprises facets formed at one end of said pinion (12), enabling this pinion (12) to engage with said tool (44).

14. Assembly according to any one of claims 1 to 13, **characterised in that** it comprises a device for assembling said implant (18a, 18b), this device comprising a base (34) provided with a housing (35), one end of which is a fixed wall (36) comprising, on the face thereof facing towards the housing (35), a horizontal crossbar (37) having a width corresponding to that of a slot (28) of the implant (18a, 18b), and the other end of which is a rod (39) movable in the longitudinal axis of the housing (35) and comprising, on the face thereof facing towards the housing (35), a crossbar (42) having a width corresponding to that of a slot (28) of the implant (18a, 18b), said crossbars (37, 42) facing one another.

15. Assembly according to any one of claims 11 to 14, **characterised in that** it comprises a tool (44) comprising a rod (45) which is provided at one of its ends with a handle (46) and at the other end with a socket (47), the internal space (48) of which is shaped so as to engage, without any possible rotation, with the posterior face (16) of the central part (11) of the distraction device (1), said rod (45) enclosing an inner rod (49) which can rotate on command about its longitudinal axis and can be immobilised in terms of rotation and comprises, at its end, means (55) for the engagement thereof with the pinion (12).

## Patentansprüche

1. Gruppe, die umfasst:
a) ein Implantat (18a, 18b), das dazu bestimmt ist, einen Wirbelkörper eines verletzten Wirbels (51) ganz oder teilweise zu ersetzen, das umfasst
- einen mittleren Teil, der ein Knochentransplantat (29) umfasst und/oder einen röhrenförmigen Käfig mit voller oder durchbrochener Wand (32),
- zwei Platten (19, 20), die an den Enden des mittleren Teils angeordnet sind, deren ebene äußeren Flächen (27) dazu bestimmt sind, mit den Wirbelplatten der gesunden Wirbel (52, 53), die den verletzten Wirbel (51) umgeben, in Kontakt zu kommen und von denen jede mit einer Kerbe (28) ausgestattet ist,
und
b) eine Distraktionsvorrichtung der Wirbelsäule (1),
wobei diese Gruppe **dadurch gekennzeichnet ist, dass** diese Distraktionsvorrichtung umfasst:
- zwei gegenüberliegende Zungen (2, 3), von denen jede eine ebene äußere Fläche (5, 6) hat, die dazu bestimmt sind, mit einer Wirbelplatte eines gesunden Wirbels (52, 53) in Kontakt zu kommen, wobei von den Zungen (2, 3) jede mit einer Zahnstange (14, 15) verbunden ist,
- einen mittleren Teil (11), der von den Zahnstangen (14, 15) durchquert wird und ein Zahnrad (12) einschließt, dessen Zähne (13) mit den Zahnstangen (14, 15) im Kontakt sind, so dass eine Drehung des Zahnrads (12) eine Annäherung oder eine relative Entfernung der Zungen (2, 3) bewirkt, und
- Mittel, um das Zahnrad (12) in Rotation zu steuern, und **dadurch**, dass von den Kerben (28) jede zur Aufnahme der Zungen (2, 3) geeignet ist, wobei die Breiten dieser Kerben (28) den Breiten dieser Zungen (2, 3) entsprechen und die Tiefe dieser Kerben (28) größer oder gleich der Dicke der Zungen (2, 3) ist.

2. Gruppe nach Anspruch 1, **dadurch gekennzeichnet, dass** von den Zungen (2, 3) jede mit den Zahnstangen (14, 15) durch einen gewundenen Abschnitt (7, 8) verbunden ist, wobei diese gewundenen Abschnitte (7, 8) derart konfiguriert sind, dass zwischen den Zungen (2, 3) und dem mittleren Teil (11) ein Versatz erfolgt, um den freien Zugang zu dem Raum hinter den Zungen (2, 3) zu erlauben.

3. Gruppe nach einem der Ansprüche 1 oder 2, **dadurch gekennzeichnet, dass** die äußeren Flächen (5) der Zungen (2, 3) parallel sind.

4. Gruppe nach Anspruch 3, **dadurch gekennzeichnet, dass** die äußeren Flächen (27) der Platten (19, 20) parallel sind.

5. Gruppe nach einem der Ansprüche 1 oder 2, **dadurch gekennzeichnet, dass** die äußeren Flächen (5, 6) der Zungen (2, 3) einen Winkel bilden, der einem Lordose- oder Kyphosewinkel der Wirbelsäule entspricht.

6. Gruppe nach Anspruch 5, **dadurch gekennzeichnet, dass** die äußeren Flächen (27) der Platten (19, 20) einen Winkel bilden, der einem Lordose- oder Kyphosewinkel der Wirbelsäule entspricht.

7. Gruppe nach einem der vorangehenden Ansprüche, **dadurch gekennzeichnet, dass** die Platten (19, 20) Durchbrüche (54) umfassen.

8. Gruppe nach einem der vorangehenden Ansprüche, **dadurch gekennzeichnet, dass** der mittlere Teil und die Platten (19, 20) ein einziges Stück bilden.

9. Gruppe nach einem der Ansprüche 1 bis 7, **dadurch gekennzeichnet, dass** die Platten (19, 20) aufgesetzt und auf dem mittleren Teil befestigt sind.

10. Gruppe nach Anspruch 9, **dadurch gekennzeichnet, dass** die Platten (19, 20) Öffnungen mit Gewinde (24, 25) für den Durchgang von Schrauben umfassen, die ihre Verbindung mit dem mittleren Teil gewährleisten.

11. Gruppe nach einem der vorangehenden Ansprüche, **dadurch gekennzeichnet, dass** die Steuermittel ein Werkzeug (44) umfassen, das imstande ist, beweglich mit dem Zahnrad (12) zusammenzuarbeiten.

12. Gruppe nach Anspruch 11, **dadurch gekennzeichnet, dass** sie einen das Zahnrad (12) durchquerenden Stift (17) umfasst, der die Ineingriffnahme dieses Zahnrads (12) mit dem Werkzeug (44) erlaubt.

13. Gruppe nach Anspruch 11, **dadurch gekennzeichnet, dass** sie in ein Ende des Zahnrads (12) eingearbeitete Flächen umfasst, die die Ineingriffnahme dieses Zahnrads (12) mit dem Werkzeug (44) erlauben.

14. Gruppe nach einem der Ansprüche 1 bis 13, **dadurch gekennzeichnet, dass** sie eine Montagevorrichtung des Implantats (18a, 18b) umfasst, wobei diese Vorrichtung einen mit einer Aufnahme (35) ausgestatteten Sockel (34) umfasst, von dem ein Ende eine starre Wand (36) ist, die auf ihrer in Richtung der Aufnahme (35) zeigenden Fläche ein horizontale Querstrebe (37) umfasst, deren Breite der Breite einer Kerbe (28) des Implantats (18a, 18b) entspricht, und dessen anderes Ende ein gemäß der Längsachse der Aufnahme (35) bewegbarer Stab (39) ist, der auf seiner in Richtung der Aufnahme (35) zeigenden Seite eine Querstrebe (42) umfasst, deren Breite der Breite einer Kerbe (28) des Implantats (18a, 18b) entspricht, wobei sich die zwei Querstreben (37, 42) gegenüberliegen.

15. Gruppe nach einem der Ansprüche 11 bis 14, **dadurch gekennzeichnet, dass** sie ein Werkzeug (44) umfasst, das einen Stab (45) aufweist, der an einem seiner Enden mit einem Griff (46) und am anderen Ende mit einer Hülse (47) ausgestattet ist, deren Innenraum (48) ausgebildet ist, um ohne mögliche Rotation mit der hinteren Seite (16) des mittleren Teils (11) der Distraktionsvorichtung (1) in Eingriff zu kommen, wobei der Stab (45) einen inneren Stab (49) einschließt, der bei Betätigung um seine Längsachse drehen und in Rotation blockiert werden kann und an seinem Ende Mittel (55) umfasst, um ihn mit dem Zahnrad (12) in Eingriff zu bringen.
